# EUROPEAN PATENT APPLICATION

(11) **EP 4 740 969 A2**
(43) Date of publication of application: **13.05.2026**
(21) Application number: 25214763.2
(22) Date of filing: 10.11.2025
(51) Int. Cl.: A61L 9/22

(54) **AIR PURIFICATION COMPONENTS, AIR CONDITIONING DEVICES**

(30) Priority: 12.11.2024 CN 202411611844
(71) Applicant: Carrier Corporation, Palm Beach Gardens, FL 33418 (US)
(72) Inventor: LI, Huanan, Shanghai, 201206 (CN); MCKINNEY, Peter Johannes, Palm Beach Gardens, 33418 (US); JIANG, Qiwen, Shanghai, 201206 (CN); JIANG, Yingchao, Shanghai, 201206 (CN); LIU, Hongsheng, Shanghai, 201206 (CN)
(74) Representative: Dehns

(57) **Abstract**

This application provides an air purification component (100) and an air conditioning device (300). The air purification component (100) includes: an outer housing (1) that is cylindrical and rotatably arranged in an air duct (301); an opening (2) provided on a cylindrical surface of the outer housing (1); an inner housing (3) extending in an axial direction of the outer housing (1) and built into the outer housing (1); an opening (4) provided on a cylindrical surface of the inner housing (3); an ionizer fixing portion (5) disposed on an inner surface of the inner housing (3); and an ionizer (6) fixed to the ionizer fixing portion (5). During operation, the ionizer (6) adsorbs dust particles in the air. In a case where the outer housing (1) pivots around a central axis thereof under the drive of wind, a surface of a carbon fiber brush is periodically wiped when the ionizer (6) transitions from the outer housing opening (2) to the inner surface of the outer housing (1), thereby cleaning the dust particles accumulated on a surface of the ionizer (6). The device according to the embodiment of this application can be driven by wind to ensure a self-cleaning function with zero energy consumption of the ionizer (6).

## Description

### PRIORITY CLAIM

This application claims benefit of Chinese Patent Application No. 202411611844.X, filed November 12, 2024, and all the benefits accruing therefrom under 35 U.S.C. §119, the contents of which in their entirety are herein incorporated by reference.

### BACKGROUND

The present invention relates to the technical field of air purification devices, in particular to an air purification component and an air conditioning device having the air purification component.

There are many problems with existing technologies and products in the field of treating chemical substances, particles, and biological contaminants in the air. The treatment cost is high, which brings about a great economic burden for practical applications. Meanwhile, the function and application range thereof are limited to some extent, and cannot fully meet the demands of different scenarios. In the aspect of a cleaning device of an air purification device, the products on the market generally need to be driven by a power device, which not only results in high energy consumption, but also results in a complex structure of the device and increases the difficulty of installation and maintenance. Moreover, the efficiency of traditional photocatalytic technologies tends to gradually decrease during use, making it difficult to maintain a stable treatment effect. In summary, current technologies and cleaning devices for treating contaminants are deficient in cost, function, efficiency, and structure.

### SUMMARY

Aspects of the present invention aim to provide an air purification component to at least solve or alleviate some of the problems existing in the related art.

It should be noted that working principles, features, advantages, and the like of the air purification component according to the present invention will be explained below by way of embodiments. However, it should be understood that all descriptions are only given for exemplification and therefore these embodiments should not be understood as forming any limitation on the present invention.

Viewed from a first aspect, there is provided an air purification component including: an outer housing that is cylindrical and rotatably arranged in an air duct; an outer housing opening provided on a cylindrical surface of the outer housing; an inner housing extending in an axial direction of the outer housing and built into the outer housing; an inner housing opening provided on a cylindrical surface of the inner housing; an ionizer fixing portion disposed on an inner surface of the inner housing; and an ionizer fixed to the ionizer fixing portion such that an end of the ionizer faces the inner surface of the inner housing.

Optionally, the ionizer is a carbon fiber brush, and the end of the ionizer extends to abut against the inner surface of the outer housing.

A plurality of carbon fiber brushes may be arranged along the axial direction of the outer housing.

The plurality of carbon fiber brushes may be additionally or alternatively uniformly arranged around the outer surface of the inner housing.

Optionally, the outer housing opening is a grille bar arranged along the axial direction of the outer housing, and the inner housing opening is a honeycomb hole arranged on the cylindrical surface of the inner housing.

An outer housing locking portion may be configured to lock and fix rotation of the outer housing such that the end of the ionizer faces the outer housing opening.

The air purification component may further include a plurality of outer housing surface blades protruding from a surface of the outer housing at a predetermined angle relative to the surface of the outer housing.

The outer housing surface blades may each be integrally formed by locally bending the outer housing.

The air purification component may further include: an inner housing catalyst coating coated on the inner surface of the inner housing; and a light emitter disposed inside the inner housing.

The light emitter may be an ultraviolet light emitter or a visible light emitter.

Viewed from a second aspect, there is provided a control device for adjusting and controlling the air purification component according to the first aspect, the control device including: an outer housing locking portion action execution module configured to activate the outer housing locking portion to lock and fix rotation of the outer housing, and allow the outer housing opening to stop at a position corresponding to an end of the ionizer.

Viewed from a third aspect, there is provided is an air conditioning device including the air purification component according the first aspect. Optionally, the air conditioning device may include the control device according to the second aspect.

### BRIEF DESCRIPTION OF THE SEVERAL VIEWS OF THE DRAWINGS

Fig. 1 is a schematic diagram of an overall structure of an air purification component.
Fig. 2 is a schematic diagram of a partial structure of the air purification component.
Fig. 3 is a schematic cross-sectional view of the air purification component shown in Fig. 1 taken along a line A-A perpendicular to a Z axis shown in the figure.
Fig. 4 is another schematic cross-sectional view of the air purification component shown in Fig. 1 taken along the line A-A perpendicular to the Z axis shown in the figure.
Fig. 5 is a schematic cross-sectional view of the air purification component shown in Fig. 1 taken along a line B-B parallel to the Z axis shown in the figure.
Fig. 6 is another schematic cross-sectional view of an air purification component 100 shown in Fig. 1 taken along the line B-B parallel to the Z axis shown in Fig. 1.
Fig. 7 is a schematic diagram of a module of an air conditioning device.

Reference numerals: air purification component 100, outer housing 1, outer housing opening 2, inner housing 3, stepped protrusion 31, inner housing opening 4, ionizer fixing portion 5, ionizer 6, hole 32, blade 7, light emitter 8, outer housing locking portion 9, and power module 10, control device 200, air conditioning device 300, air duct 301.

### DETAILED DESCRIPTION OF THE DISCLOSURE

It should be noted that working principles, features, advantages, and the like of the air purification component according to the present invention will be discussed below. However, it should be understood that all descriptions are only given for exemplification and should not be understood as forming any limitation on the scope of the present invention, which is defined by the appended claims.

In addition, for any single technical feature described or implicit in some embodiments mentioned herein, or any single technical feature shown or implicit in the drawings, the disclosure includes any combination or deletion between these technical features (or their equivalents) without any technical obstacles, thereby obtaining other embodiments that may not be directly mentioned herein.

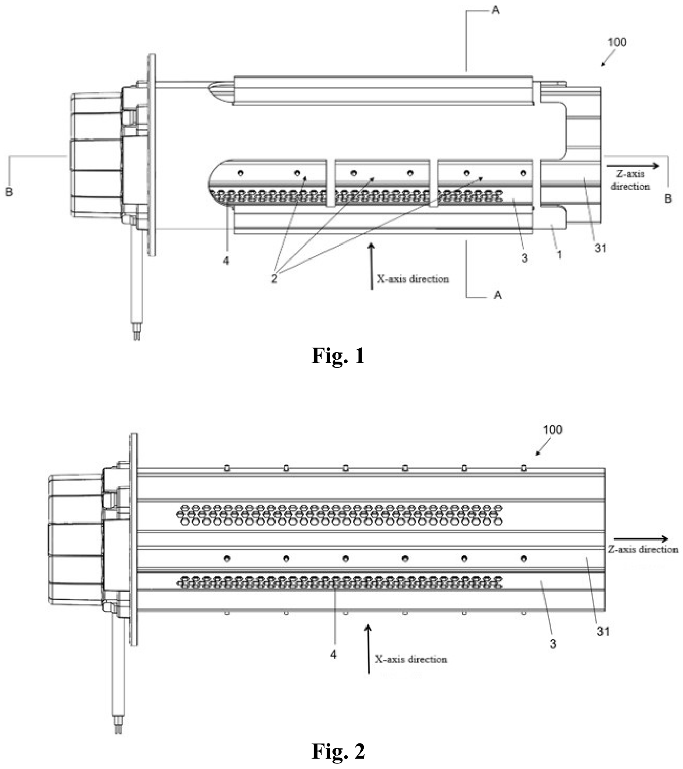

Fig. 1 is a schematic diagram of an overall structure of an air purification component 100 according to one or more embodiments. As shown in Fig. 1, the air purification component 100 includes an outer housing 1 extending in a Z-axis direction shown in the figure and substantially cylindrical, and an inner housing 3 surrounded by the outer housing 1 and extending in the same axial direction as the outer housing 1, and the outer housing 1 can pivot around a central axis thereof. The inner housing 3 is substantially cylindrical as a whole, and a plurality of stepped protrusions 31 extending in the Z-axis direction as shown in the figure are provided at equal intervals along a surface of the inner housing 3. A certain gap is left between an outer surface of the inner housing 3 and an inner surface of the outer housing 1, so that the outer housing 1 does not contact the outer surface of the inner housing 3 when pivoting about the central axis. A plurality of outer housing openings 2 are circumferentially arranged around a cylindrical surface of the outer housing 1.

Fig. 2 is a schematic diagram of a partial structure of the air purification component 100 according to the embodiment. As shown in Fig. 2, a plurality of inner housing openings 4 extending in the Z-axis direction shown in the figure are circumferentially arranged around the surface of the inner housing 3. The inner housing openings 4 and the stepped protrusions 31 of the inner housing 3 are both arranged around the surface of the inner housing 3, and the inner housing openings 4 and the stepped protrusions 31 of the inner housing 3 are arranged at intervals in a circumferential direction of the surface of the inner housing 3.

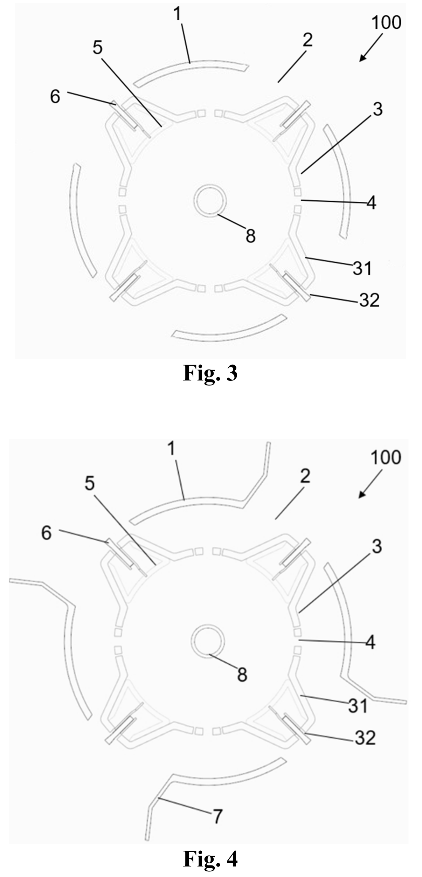

Fig. 3 is a schematic cross-sectional view of the air purification component 100 shown in Fig. 1 taken along a line A-A perpendicular to a Z axis shown in Fig. 1. As shown in Fig. 3, corresponding to the stepped protrusions 31 extending in the Z-axis direction shown in Fig. 1, a plurality of ionizer fixing portions 5 are arranged, and the ionizer fixing portions 5 are accommodated in the stepped protrusions 31 of the inner housing 3 in a shape-matched manner and are spaced apart from each other at a predetermined interval in the circumferential direction of the inner housing. One end of an ionizer 6 close to the central axis is fixed to each of the ionizer fixing portions 5, one end of the ionizer 6 away from the central axis extends toward the inner surface of the outer housing 1, and a hole 32 is provided at a position corresponding to a surface of each of the stepped protrusions 31 of the inner housing 3, so that one end of the ionizer 6 away from the central axis can pass through the hole 32 and extend out of an outer surface of the stepped protrusion 31 of the inner housing 3, and one end of the ionizer 6 away from the central axis faces the inner surface of the outer housing 1.

The air purification component 100 is arranged in an air duct 301 such that the Z axis shown in Fig. 1 is substantially perpendicular to an airflow direction (X direction shown in the figure).

When the air flows in the X direction shown in the figure, part of the airflow enters the cylindrical outer housing 1 from the outer housing opening 2, so that part of the wind pressure inside the outer housing 1 becomes uneven, resulting in asymmetric pressure distribution, and then a pressure difference is formed to cause the outer housing 1 to pivot around the central axis under the drive of wind. During operation, the ionizer 6 is energized, so that the end of the ionizer 6 carries a negative charge and absorbs dust particles flowing in the air, thereby purifying the air. In a case where the outer housing 1 pivots around the central axis and the inner housing 3 remains fixed, with the rotation of the outer housing 1, when the outer housing opening 2 rotates to the end of the ionizer 6, dust particles and other substances in the air flowing into the air purification component 100 through the outer housing opening 2 are adsorbed by the ionizer 6; when the outer housing opening 2 transitions to the inner surface of the outer housing 1, the ionizer 6 can abut against an end of the inner surface of the outer housing 1 and be periodically wiped by the inner surface of the outer housing 1, so as to clean the dust particles adsorbed by the end of the ionizer 6 and maintain the effect of the ionizer 6 being charged to adsorb dust particles.

At the same time, since the inner housing opening 4 is provided on the surface of the inner housing 3, the part of the airflow entering the interior of the outer housing 1 does not create excessive resistance and significantly increase wind resistance in the air duct 301 due to the presence of the air purification component 100.

In some embodiments, there is no need to configure a drive device for the air purification component 100, but the outer housing 1 is pivoted around the central axis by means of wind. When the outer housing opening 2 rotates to the end of the ionizer 6, the dust particles and other substances in the air flowing into the air purification component 100 through the outer housing opening 2 are adsorbed by the ionizer 6, and when the outer housing opening 2 transitions to the inner surface of the outer housing 1, the ionizer 6 can abut against an end of the inner surface of the outer housing 1 and be periodically wiped by the outer housing 1, so as to achieve zero-energy consumption and self-cleaning effects. Wind energy is utilized to ensure the self-cleaning function of the ionizer 6, thereby improving the cleaning efficiency of air purification. In addition, the air purification component has a simple structure and saves energy.

As an embodiment, the ionizer 6 is configured as a carbon fiber brush, and one end of the ionizer 6 away from the central axis extends to abut against the inner surface of the outer housing 1.

The carbon fiber has strong conductivity, wear resistance and portability in a filament direction thereof, and a diameter of a carbon fiber filament is extremely small. By using the carbon fiber brush as the ionizer 6, a plurality of carbon fiber filaments can be uniformly distributed in each ionizer 6. Meanwhile, the ionizer 6 can absorb dust particles in the air more firmly through the strong conductivity of the carbon fiber, thereby reducing the possibility that the dust particles absorbed on the ionizer 6 are blown away from the ionizer 6 and back to the air due to a high speed or large force of the wind passing through the air purification component 100. At the same time, one end of the ionizer 6 away from the central axis extends to abut against the inner surface of the outer housing 1, and thus, when the outer housing 1 pivots around the central axis under the action of wind, the inner surface of the outer housing 1 can directly wipe the ionizer 6. Since the carbon fiber brush has good wear resistance and portability, the carbon fiber brush is used as the ionizer 6, which avoids the problem that the inner surface of the outer housing 1 is in direct contact with the ionizer 6 for a long time and intermittently, which may cause wear on the outer housing 1 or bending of the ionizer 6, and also avoids the possibility that when other materials with poor portability are used as the ionizer 6, the force of the wind flowing through the air purification component 100 is small, which causes a reaction force on the rotation of the outer housing 1.

The air purification component 100 according to the embodiment is described by taking the example that the ionizer fixing portion 5 is arranged at the stepped protrusion 31 in a matched manner, but is not limited thereto. Any structural form capable of fixing the ionizer 6 to the inner housing 3 can achieve the technical effects of this application and falls within the protection scope of this application. Similarly, the shapes and quantities of the stepped protrusion 31 and the ionizer fixing portion 5 are not limited in some embodiments. The quantity and distribution of the ionizers 6 can be flexibly arranged by those skilled in the art according to the shape and structure of the ionizer fixing portion 5, and all such configurations can achieve the technical effects of this application and fall within the protection scope of this application.

In addition, it should be noted that the shape and quantity of the outer housing opening 2 are not limited in some embodiments. Any configuration that utilizes wind force to allow airflow to enter the cylindrical outer housing 1 through the outer housing opening 2, thereby enabling the outer housing 1 to pivot around the central axis, falls within the protection scope of this application.

In one or more embodiments, the outer housing opening 2 is configured as a grille bar arranged along an axial direction of the outer housing 1.

Through the above embodiment, the outer housings 1 on both sides of the outer housing opening 2 are connected by the grille bar, so that the outer housings 1 on both sides of the outer housing opening 2 interact with each other, thereby reducing the possibility that deformation of the outer housing 1 due to wind or long-term gravity effects impacts the rotation around the central axis of the outer housing 1.

In one or more embodiments, the inner housing opening 4 is a honeycomb hole provided on a cylindrical surface of the inner housing 3.

When the outer housing 1 rotates to a position abutting against the ionizer 6, the ionizer 6 cannot continue to adsorb impurities in the air. By providing the honeycomb hole, the air flowing into the inner housing 3 is initially filtered to prevent large impurities from flowing into the inner housing 3. When the air flows to a leeward side of the inner housing, the dust particles in the air are adsorbed by the ionizer 6 arranged in a leeward direction.

Similarly, although the honeycomb hole is taken as an example to illustrate the inner housing opening 4, the specific shape of the inner housing opening 4 is not limited. For example, any shape, such as the shape of the grille or narrow slit, that can achieve the technical effects of this application should fall within the protection scope of this application.

As an embodiment, on the basis of the above, a plurality of carbon fiber brushes may be provided to improve the cleaning efficiency, and two solutions for providing a plurality of carbon fiber brushes are given below.

First solution: A plurality of carbon fiber brushes are arranged along the Z-axis direction shown in Fig. 1 on the outer surface of the inner housing 3.

The plurality of carbon fiber brushes arranged along the Z-axis direction shown in Fig. 1 on the outer surface of the inner housing 3 adsorb dust particles in the air flowing through different positions of the air purification component 100, thereby avoiding the problem of affecting the purification effect on the air flowing through other parts due to the concentrated arrangement in a certain part of the air purification component 100.

Second solution: A plurality of carbon fiber brushes are uniformly arranged around the outer surface of the inner housing 3.

The plurality of carbon fiber brushes are uniformly arranged around the outer surface of the inner housing 3, so that a purification range of the air purification component 100 is expanded, and dust particles in the air flowing through the outside of the inner housing 3 can also be adsorbed, thereby improving air purification efficiency. At the same time, when the air flows to the leeward side of the inner housing, dust particles that cannot be completely adsorbed in the air can still be re-adsorbed by the carbon fiber brush arranged in the leeward direction.

It should be noted that the ionizer 6 in some embodiments is not limited to the carbon fiber brush, and other materials with good conductivity, adsorption performance, wear resistance and the like that can be used in conjunction with this application are within the protection scope of this application. In addition, although the solutions of providing a plurality of carbon fiber brushes are illustrated by way of example in some embodiments, those skilled in the art may set solutions of providing a plurality of carbon fiber brushes according to other situations such as the shape and structure of the outer housing 1 and the inner housing 3, including but not limited to the above two solutions and combinations thereof. The situation in which the solutions differ from one another also falls within the protection scope of this application.

In an air purification component according to a second embodiment, elements denoted by the same names and symbols as those of the air purification component 100 in the above embodiment of this application represent identical components, and descriptions thereof are omitted here.

Fig. 4 is another schematic cross-sectional view of the air purification component 100 shown in Fig. 1 taken along the line A-A perpendicular to the Z axis shown in Fig. 1 according to the embodiment. As shown in Fig. 4, in order to improve the utilization effect of wind energy, unlike the first embodiment, a plurality of outer housing surface blades 7 extending in the Z-axis direction shown in Fig. 1 are provided on the surface of the outer housing 1 of the air purification component 100 according to the second embodiment of this application. Specifically, the outer housing surface blades 7 protrude from the surface of the outer housing 1 at a predetermined angle (cut angle) relative to the surface of the outer housing 1, and thus, when the air flows in the X-axis direction shown in the figure, the torque of rotation around the central axis can be generated more advantageously by means of wind, thereby improving the efficiency of the outer housing 1 pivoting around the central axis, and indirectly improving the wiping frequency and force of the ionizer 6 (carbon fiber brush) abutting against the end of the inner surface of the outer housing 1.

Further, the outer housing surface blades 7 may be connected to a cross section of the outer housing opening 2 extending in the Z-axis direction shown in Fig. 1 and integrally formed with the outer housing 1, and the outer housing surface blades 7 each are formed by partially bending the outer housing 1 at a predetermined angle.

It should be noted that, in some embodiments, the quantity, shape and bending angle of the outer housing surface blades 7 and the relative positional relationship between the outer housing surface blades 7 and the surface of the outer housing 1 are not limited. Any shape, structure and quantity of the outer housing surface blades 7 that improve the wind energy conversion efficiency of the outer housing 1 pivoting around the central axis can achieve the technical effects of this application and fall within the protection scope of this application.

Further, although this embodiment has been described in the form in which the outer housing surface blades 7 protrude from the surface of the outer housing 1, it is not limited thereto. The outer housing surface blades 7 may also be blades extending toward the inner surface of the outer housing 1, as long as the blades do not contact the outer surface of the inner housing 3 and can generate the torque for rotating the outer housing 1 around the central axis by means of part of the wind flowing into the interior of the outer housing 1. All such configurations fall within the protection scope of this application.

In an air purification component according to a third embodiment, elements denoted by the same names and symbols as those of the air purification component 100 in the above embodiment of this application represent identical components, and descriptions thereof are omitted here.

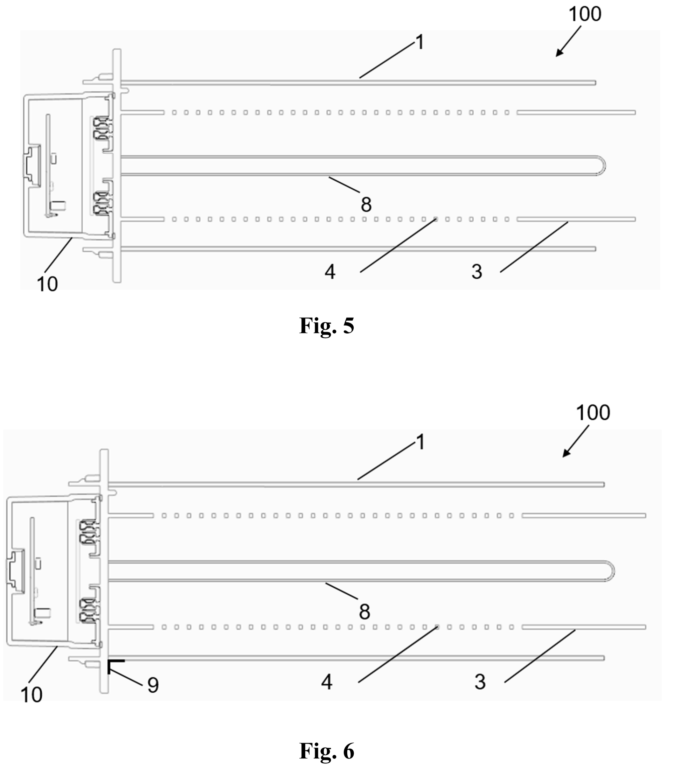

Fig. 5 is a schematic cross-sectional view of the air purification component 100 shown in Fig. 1 taken along a line B-B parallel to the Z axis shown in Fig. 1. As shown in Fig. 5, the third embodiment is based on all the above embodiments, and in order to further improve the air purification effect, a linear light emitter 8 extending along the Z-axis direction shown in Fig. 1 is arranged at a central position inside the inner housing 3, and is preferably an ultraviolet lamp, a visible light, and the like.

At the same time, the inner surface of the inner housing 3 is coated with a catalyst coating, such as TiO2 and graphene-TiO2, which can be irradiated at multiple angles by the light emitter 8 and can be used for sterilization and removal of TVOC. It should be noted that, although the above embodiment has been described in the form in which the linear light emitter 8 is arranged at the central position inside the inner housing 3 and extends along the Z-axis direction shown in Fig. 1, the shape of the light emitter 8 and the position where the light emitter 8 is arranged in the inner housing 3, and the type and coverage area of the catalyst coating are not specifically limited in some embodiments. Any configuration in which the light emitter is arranged in the inner housing 3 and a sterilization effect of the light emitter 8 can be enhanced by any type of catalyst can achieve the technical effects of this application and falls within the protection scope of this application.

Preferably, in the air purification component 100 in the above embodiment, a flow direction of the air (X direction shown in Fig. 1) is substantially perpendicular to the Z axis shown in Fig. 1.

According to this embodiment, in a case where the air flows to the air purification component 100 from the X direction shown in the figure, when the air enters from the outer housing opening 2, and then flows through one end of the ionizer 6 away from the central axis, the end of the ionizer 6 is negatively charged in the energized state and effectively adsorbs and removes dust particles in the air. When the airflow further flows from the inner housing opening 4 into the inner housing 3 and contacts the catalyst coating on the inner surface of the inner housing 3 irradiated by the light emitter, the activity of the catalyst is maximally stimulated by irradiating the catalyst coating at multiple angles with the light emitter 8, promoting the catalyst to continuously generate active oxygen species to undergo oxidation reactions with TVOC in the air and decompose pollutants in the air, thereby effectively reducing the concentration of the pollutants in the air. At the same time, the airflow is irradiated by the light emitter 8 built in the inner housing 3, and the light emitter 8 can effectively kill bacteria, viruses and other microorganisms in the airflow. In this way, the air purification effect is achieved.

In an air purification component according to a fourth embodiment, elements denoted by the same names and symbols as those of the air purification component 100 in the above embodiment represent identical components, and descriptions thereof are omitted here.

Fig. 6 is another schematic cross-sectional view of the air purification component 100 shown in Fig. 1 taken along the line B-B parallel to the Z axis shown in Fig. 1 according to the embodiment. As shown in Fig. 6, as an embodiment, in order to more flexibly control the process of adsorbing dust particles by the ionizer 6 in the air purification component 100, an outer housing locking portion 9 for locking and fixing the rotation of the outer housing 1 in a specific orientation is provided in the air purification component 100 in this application. Specifically, the outer housing locking portion 9 is a pin device, a pin base of the outer housing locking portion 9 is fixed to a side plate surface of a power module 10 close to the outer surface of the outer housing 1, and a pin rod and a pin head of the outer housing locking portion 9 are fixed on an outer surface of the outer housing 1 close to one end of the power module 10. In a case where it is determined that there are few dust particles in the air, that is, the ionizer 6 does not need to be cleaned frequently through the outer housing 1, when the end of the ionizer 6 faces the outer housing opening 2, the pin rod of the outer housing locking portion 9 is fixed to the pin base matched with the pin rod so as to maintain the state in which the outer housing 1 stops rotating and remains stationary in a specific orientation under windy conditions.

More specifically, the outer housing locking portion 9 has two states: open and closed. When the outer housing locking portion 9 is opened, the outer housing pivots about the central axis, and the end of the ionizer 6 abutting against the inner surface of the outer housing 1 adsorbs dust particles in the air when passing through the outer housing opening 2, and is wiped when transitioning from the outer housing opening 2 to the inner surface of the outer housing 1, so that the ionizer 6 realizes a periodic self-cleaning function. When the outer housing locking portion 9 is closed, the outer housing 1 is fixed from a rotating state to a stationary state with the end of the ionizer 6 facing the outer housing opening 2. By fixing the rotation of the outer housing in a specific orientation, the ionizer in this state continuously adsorbs dust particles in the air without being periodically cleaned, thereby making the control of the air purification component 100 more flexible.

Further, the pin device referred to in this application may preferably be a retractable pin utilizing an electromagnetic adsorption force. The pin is retracted using the electromagnetic adsorption force when energized, so that the outer housing 1 can rotate freely. During the power outage, the electromagnetic force disappears, the pin extends to block the free rotation of the outer housing 1, so that the outer housing 1 can stay at a preset position. It should be noted that the above is merely an example of the pin device, and does not limit a specific structure of the pin device.

Although the outer housing locking portion 9 provided in some embodiments is described by taking the pin device as an example, it is not limited thereto. Any device that can control the outer housing 1 to stop rotating with the end of the ionizer 6 facing the outer housing opening 2 can achieve the technical effects of this application and falls within the protection scope of this application. The specific shape, structure, quantity and position of the outer housing locking portion 9 and the specific angle at which the end of the ionizer 6 faces the outer housing opening 2 are not limited in some embodiments.

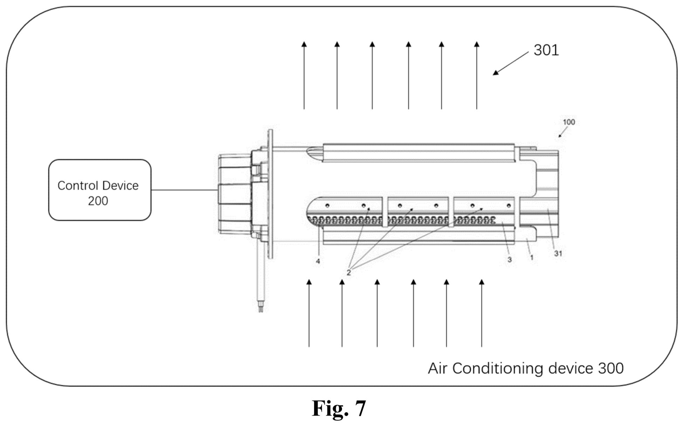

In addition, Fig. 7 is a schematic diagram of a module of an air conditioning device provided in one or more embodiments. As shown in Fig. 6, one or more embodiments may also provide a control device 200 for adjusting and controlling the air purification component 100 according to the embodiment, and the control device 200 includes an outer housing locking portion action execution module.

When it is determined that there are few dust particles in the air, that is, the ionizer 6 does not need to be cleaned frequently through the outer housing 1, the outer housing locking portion action execution module is executed to activate the outer housing locking portion 9 to lock and fix the rotation of the outer housing 1, so that the outer housing 1 is fixed from a rotating state to a stationary state with the end of the ionizer 6 facing the outer housing opening 2. By fixing the rotation of the outer housing in a specific orientation, the ionizer in this state continuously adsorbs dust particles in the air without being periodically cleaned, thereby making the control of the air purification component 100 more flexible.

In addition, an embodiment may also provide an air conditioning device 300, and for example, the air conditioning device 300 may be an air purification device or an air conditioner. The air conditioning device 300 according to the embodiment is provided with the air purification component 100 according to any one of the above embodiments in an air duct 301 therein.

Therefore, when the air purification component 100 is arranged in the air duct 301 of the air conditioning device 300 such that the Z axis shown in Fig. 1 is substantially perpendicular to the flow direction of the air (X direction shown in Fig. 1), the air conditioning device 300 can adsorb and remove dust particles in the air through the ionizer 6, and undergo an oxidation reaction with the catalyst coating on the inner surface of the inner housing 3 irradiated by the light emitter 8 to reduce the concentration of pollutants in the airflow. Meanwhile, the airflow is irradiated by the light emitter 8 to kill bacteria, viruses and other microorganisms in the airflow, and the air is continuously circulated back and forth to achieve the air purification effect.

In the above process, the outer housing 1 periodically wipes the dust particles accumulated at the end of the ionizer 6 abutting against the inner surface of the outer housing when the outer housing 1 is driven by the wind in the air duct 301 to pivot around the central axis, thereby avoiding the situation that the adsorption efficiency is reduced due to excessive dust particle accumulation on the surface of the ionizer 6. The ionizer 6 is self-cleaned with zero energy consumption by means of wind drive, so that the ionizer 6 can be cleaned and maintained without configuring a drive device for the air purification component 100 in the air conditioning device 300, thereby achieving an energy-saving air purification effect.

The specific embodiments described above are only used to more clearly describe the principles of the present invention, and various components are clearly illustrated or described to make the principles of the present invention easier to understand. Those skilled in the art can easily make various modifications or changes to the specific embodiments described above without departing from the scope of this invention as defined by the scope of the appended claims.

## Claims

1. An air purification component (100), comprising:
an outer housing (1) that is cylindrical and rotatably arranged in an air duct (301);
an outer housing opening (2) provided on a cylindrical surface of the outer housing (1);
an inner housing (3) extending in an axial direction of the outer housing (1) and built into the outer housing (1);
an inner housing opening (4) provided on a cylindrical surface of the inner housing (3);
an ionizer fixing portion (5) disposed on an inner surface of the inner housing (3); and
an ionizer (6) fixed to the ionizer fixing portion (5) such that an end of the ionizer (6) faces the inner surface of the inner housing (3).

2. The air purification component (100) according to claim 1, wherein the ionizer (6) is a carbon fiber brush, and the end of the ionizer (6) extends to abut against the inner surface of the outer housing (1).

3. The air purification component (100) according to claim 2, wherein a plurality of carbon fiber brushes are arranged along the axial direction of the outer housing (1).

4. The air purification component (100) according to claim 2 or 3, wherein a plurality of carbon fiber brushes are uniformly arranged around the outer surface of the inner housing (1).

5. The air purification component (100) according to any preceding claim, wherein
the outer housing opening (2) is a grille bar arranged along the axial direction of the outer housing (1), and
the inner housing opening (4) is a honeycomb hole arranged on the cylindrical surface of the inner housing (3).

6. The air purification component (100) according to claim 5, further comprising:
an outer housing locking portion (9) configured to lock and fix rotation of the outer housing (1) such that the end of the ionizer (6) faces the outer housing opening (2).

7. The air purification component (100) according to any preceding claim, further comprising:
a plurality of outer housing surface blades (7) protruding from a surface of the outer housing (1) at a predetermined angle relative to the surface of the outer housing (1).

8. The air purification component (100) according to claim 7, wherein the outer housing surface blades (7) each are integrally formed by locally bending the outer housing (1).

9. The air purification component (100) according to any preceding claim, further comprising:
an inner housing catalyst coating coated on the inner surface of the inner housing (3); and
a light emitter (8) disposed inside the inner housing (3).

10. The air purification component (100) according to claim 9, wherein the light emitter (8) is an ultraviolet light emitter.

11. The air purification component (100) according to claim 9, wherein the light emitter (8) is a visible light emitter.

12. A control device (200) for adjusting and controlling the air purification component (100) according to claim 6, the control device (200) comprising:
an outer housing locking portion action execution module configured to activate the outer housing locking portion (9) to lock and fix rotation of the outer housing (1), and allow the outer housing opening (2) to stop at a position corresponding to an end of the ionizer (6).

13. An air conditioning device (300) comprising: an air purification component (100) according to any of claims 1 to 12.
